# EUROPEAN PATENT APPLICATION

(11) **EP 3 121 785 A1**
(43) Date of publication of application: **25.01.2017**
(21) Application number: 14886648.6
(22) Date of filing: 16.12.2014
(51) Int. Cl.: G06Q 50/10, G06Q 50/00

(54) **SCHEME RECOMMENDATION DEVICE, SCHEME RECOMMENDATION METHOD, AND SCHEME RECOMMENDATION PROGRAM**

(30) Priority: 19.03.2014 JP 2014055840
(71) Applicant: NEC Solution Innovators, Ltd., Tokyo 136-8627 (JP)
(72) Inventor: TAGUCHI, Takehiro, Tokyo 136-8627 (JP)
(74) Representative: Vossius & Partner Patentanwälte Rechtsanwälte mbB
(86) International application number: PCT/JP2014/006265
(87) International publication number: WO 2015/140860

(57) **Abstract**

A scheme result determination unit 81 determines, for each scheme executed by each user, whether the execution of the scheme by the user is successful or failed, based on a duration of the execution of the scheme by the user and a duration threshold or range used to determine whether or not the scheme is successful. A combination generation unit 82 generates, for each user, a combination including a failed scheme and a successful scheme based on a result of the determination, the failed scheme being a scheme determined as failed, and the successful scheme being a scheme determined as successful. A scheme recommendation unit 83, when a scheme recognized as failed is input, extracts a combination including the failed scheme that matches the input scheme from the generated combinations, and recommends the successful scheme included in the extracted combination.

## Description

### Technical Field

The present invention relates to a scheme recommendation device, scheme recommendation method, and scheme recommendation program for recommending an appropriate scheme to a person who has failed to execute a scheme.

### Background Art

In cognitive behavioral therapy (CBT), counseling or the like is provided based on a task for behavior modification and a scheme for performing the task. For example, in cognitive behavioral therapy for insomnia (CBT-I), a client is given a task every day for one month, and continuously performs the given task to modify his or her behaviors. Thus, tasks or schemes recommended to clients are important elements in CBT.

As a typical technique of recommending information to users, Non Patent Literature (NPL) 1 describes collaborative filtering recommendation. How to recommend desired products to potential customers is crucial for advertisers. In collaborative filtering recommendation, the preference patterns of Internet users are learned based on the users' site browsing histories, click histories, etc., and products which the users may like are recommended to them.

### Citation List

### Non Patent Literature

NPL 1: "Collaborative Filtering Recommendation", March 28, 2003, Japan Patent Office, [searched on February 24, 2014], Internet <URL: http://www.jpo.go.jp/shiryou/s_sonota/hyoujun_gijutsu/net_koukoku/134.html>

### Summary of Invention

### Technical Problem

In the case of keeping the activity of improving lifestyle as in the aforementioned CBT, it is important to recommend such a scheme that is suitable for the person so that the person can continue the scheme without getting tired of it, depending on the person's personality and the activity.

The collaborative filtering recommendation described in NPL 1 is a technique focused on encouraging users to start using new services or buy new products. In detail, the technique described in NPL 1 recommends, based on users' past preferences, the same or similar products, etc. to other users.

In a situation where such a scheme that requires continuity is executed, it is preferable to appropriately recommend, to a person who has failed to successfully execute a scheme, the next best scheme by making use of the fact that the person has failed to successfully execute the scheme.

The present invention accordingly has an object of providing a scheme recommendation device, scheme recommendation method, and scheme recommendation program for appropriately recommending, to a person who has failed to successfully execute a scheme, the next best scheme.

### Solution to Problem

A scheme recommendation device according to the present invention includes: a scheme result determination unit which determines, for each scheme executed by each user, whether the execution of the scheme by the user is successful or failed, based on a duration of the execution of the scheme by the user and a duration threshold or range used to determine whether or not the scheme is successful; a combination generation unit which generates, for each user, a combination including a failed scheme and a successful scheme based on a result of the determination, the failed scheme being a scheme determined as failed, and the successful scheme being a scheme determined as successful; and a scheme recommendation unit, when a scheme recognized as failed is input, which extracts extracting a combination including the failed scheme that matches the input scheme from generated combinations, and recommends the successful scheme included in the extracted combination.

A scheme recommendation method according to the present invention is performed by a scheme recommendation device, and includes: determining, for each scheme executed by each user, whether the execution of the scheme by the user is successful or failed, based on a duration of the execution of the scheme by the user and a duration threshold or range used to determine whether or not the scheme is successful; generating, for each user, a combination including a failed scheme and a successful scheme based on a result of the determination, the failed scheme being a scheme determined as failed, and the successful scheme being a scheme determined as successful; and extracting, when a scheme recognized as failed is input, a combination including the failed scheme that matches the input scheme from generated combinations, and recommending the successful scheme included in the extracted combination.

A scheme recommendation program according to the present invention causes a computer to execute: a scheme result determination process of determining, for each scheme executed by each user, whether the execution of the scheme by the user is successful or failed, based on a duration of the execution of the scheme by the user and a duration threshold or range used to determine whether or not the scheme is successful; a combination generation process of generating, for each user, a combination including a failed scheme and a successful scheme based on a result of the determination, the failed scheme being a scheme determined as failed, and the successful scheme being a scheme determined as successful; and a scheme recommendation process of, when a scheme recognized as failed is input, extracting a combination including the failed scheme that matches the input scheme from generated combinations, and recommending the successful scheme included in the extracted combination.

### Advantageous Effects of Invention

According to the present invention, it is possible to appropriately recommend, to a person who has failed to successfully execute a scheme, the next best scheme.

### Brief Description of Drawings

[Fig. 1] It depicts a block diagram depicting an example of the structure of Exemplary Embodiment 1 of a scheme recommendation device according to the present invention.
[Fig. 2] It depicts an explanatory diagram depicting an example of information stored in a scheme history storage unit.
[Fig. 3] It depicts an explanatory diagram depicting an example of schemes.
[Fig. 4] It depicts an explanatory diagram depicting an example of criteria.
[Fig. 5] It depicts an explanatory diagram depicting an example of the process of determining whether each scheme is successful or failed.
[Fig. 6] It depicts an explanatory diagram depicting an example of the process of generating combinations of failed schemes and successful schemes.
[Fig. 7] It depicts an explanatory diagram depicting an example of the process of recommending one or more schemes.
[Fig. 8] It depicts a flowchart depicting an example of the operation of the scheme recommendation device in Exemplary Embodiment 1.
[Fig. 9] It depicts a block diagram depicting an example of the structure of Exemplary Embodiment 2 of a scheme recommendation device according to the present invention.
[Fig. 10] It depicts an explanatory diagram depicting a first example of the process of summarizing combinations.
[Fig. 11] It depicts an explanatory diagram depicting a second example of the process of summarizing combinations.
[Fig. 12] It depicts an explanatory diagram depicting a third example of the process of summarizing combinations.
[Fig. 13] It depicts a flowchart depicting an example of the operation of the scheme recommendation device in Exemplary Embodiment 2.
[Fig. 14] It depicts a block diagram depicting an example of the structure of Exemplary Embodiment 3 of a scheme recommendation device according to the present invention.
[Fig. 15] It depicts an explanatory diagram depicting an example of categories.
[Fig. 16] It depicts an explanatory diagram depicting an example of adding categories to combinations of successful schemes and failed schemes.
[Fig. 17] It depicts an explanatory diagram depicting another example of recommending one or more schemes.
[Fig. 18] It depicts a block diagram depicting an example of the structure of Exemplary Embodiment 4 of a scheme recommendation device according to the present invention.
[Fig. 19] It depicts an explanatory diagram depicting an example of combinations to which attribute information has been added.
[Fig. 20] It depicts an explanatory diagram depicting another example of recommending one or more schemes.
[Fig. 21] It depicts a block diagram schematically depicting a scheme recommendation device according to the present invention.

### Description of Embodiment

The following describes exemplary embodiments of the present invention with reference to drawings.

### Exemplary Embodiment 1

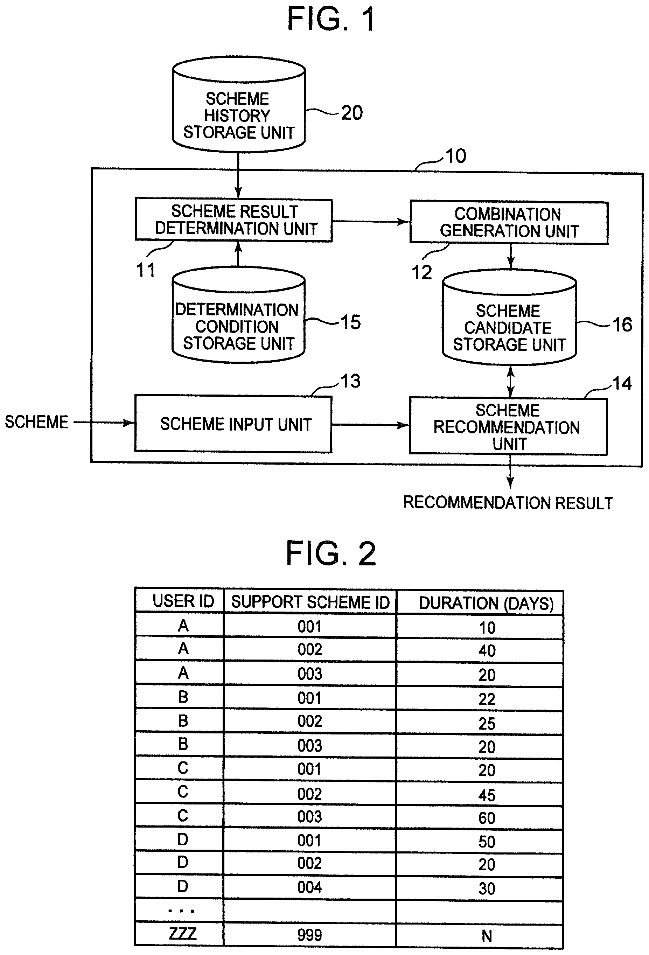

Fig. 1 is a block diagram depicting an example of the structure of Exemplary Embodiment 1 of a scheme recommendation device according to the present invention. A scheme recommendation device 10 in this exemplary embodiment includes a scheme result determination unit 11, a combination generation unit 12, a scheme input unit 13, a scheme recommendation unit 14, a determination condition storage unit 15, and a scheme candidate storage unit 16. The scheme recommendation device 10 may also include a scheme history storage unit 20 described later.

In the following description, a person who executes a scheme is referred to as "user". The scheme recommendation device according to the present invention may be used by the user for the purpose of being provided with a recommendation of a new scheme. Moreover, a person who determines whether or not the execution of the scheme by the user is failed and recommends the next best scheme to the user is referred to as "supervisor". The scheme recommendation device according to the present invention may be used by the supervisor who determines whether the execution of the scheme by the user is successful or failed, for the purpose of recommending the next best scheme to the user.

The scheme history storage unit 20 stores information to be determined by the scheme result determination unit 11. In detail, the scheme history storage unit 20 stores the duration of each scheme executed by each user. In the following description, information indicating the duration of each scheme executed by each user is also referred to as "execution history".

Fig. 2 is an explanatory diagram depicting an example of the information stored in the scheme history storage unit 20. In the example depicted in Fig. 2, the scheme history storage unit 20 stores, for each user ID identifying a user, a support scheme ID identifying a scheme executed by the user and a duration indicating the execution period of the scheme. In the example depicted in Fig. 2, the number of days is set as the duration. However, the information indicating the duration is not limited to the number of days, and may be the number of months, the number of years, time, or the like. The scheme history storage unit 20 may store not only the duration but also other information in association with the scheme executed by the user.

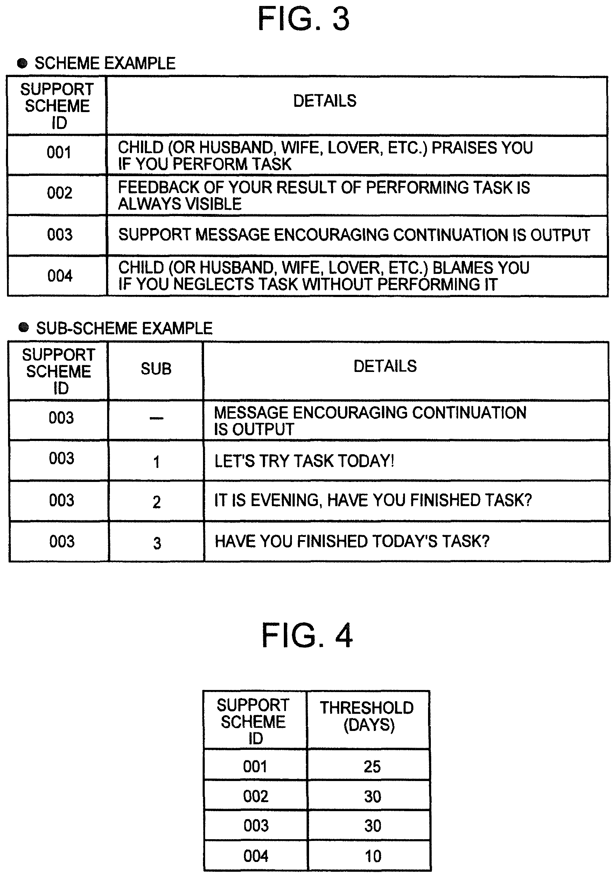

Fig. 3 is an explanatory diagram depicting an example of schemes. In the example depicted in Fig. 3, the support scheme IDs and the scheme details are managed in association with each other. In this exemplary embodiment, there are two or more types of schemes. A scheme identified by a support scheme ID may be associated with a plurality of sub-schemes for realizing the scheme, as depicted in Fig. 3. In the example depicted in Fig. 3, the scheme identified by the support scheme ID "003" is associated with three sub-schemes. The scheme recommendation device 10 may, when a support scheme ID is designated, determine whether or not the scheme has sub-schemes.

The scheme result determination unit 11 determines, for each scheme executed by each user, whether the execution of the scheme by the user is successful or failed, based on the duration of the execution of the scheme by the user. Here, the scheme result determination unit 11 determines whether the execution of the scheme by the user is successful or failed, based on a duration criterion used to determine whether or not the scheme is successful. In the example depicted in Fig. 1, the duration criterion is stored in the determination condition storage unit 15. The duration criterion may be set for each scheme, or one duration criterion may be set for the whole processes. In the case where the duration criterion is set for each scheme, finer determination is possible. In the case where one duration criterion is set for the whole processes, the number of items set by the user or the supervisor is reduced, which contributes to lower setting complexity.

Fig. 4 is an explanatory diagram depicting an example of the criteria stored in the determination condition storage unit 15. In the example depicted in Fig. 4, the determination condition storage unit 15 stores, for each scheme, a threshold of the duration with which the scheme is determined as successful, as the duration criterion. In the case where the duration of the execution of the scheme by the user is not less than the set threshold, the scheme result determination unit 11 determines that the execution of the scheme is successful. The determination condition storage unit 15 is realized by memory as an example.

The criterion used for the determination by the scheme result determination unit 11 is not limited to the duration threshold. For example, the scheme result determination unit 11 may use a range of the duration with which the scheme is determined as successful, as the criterion.

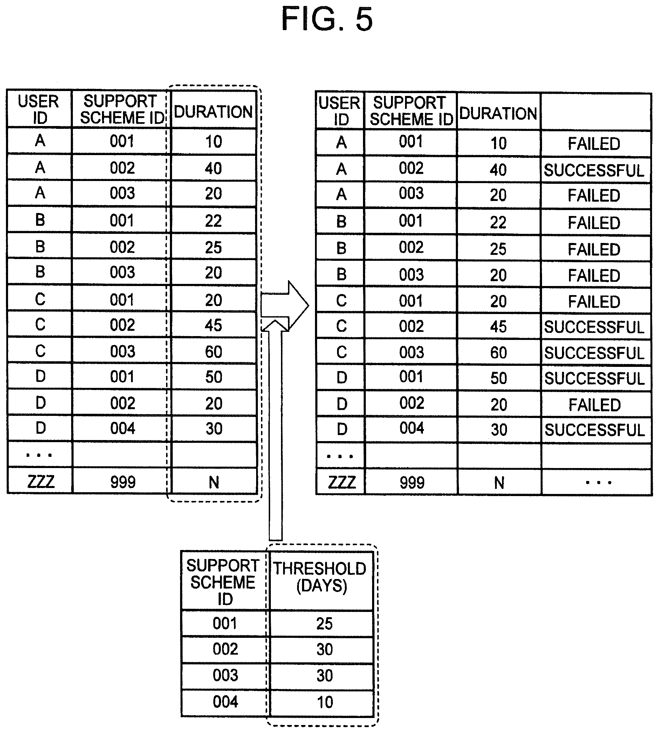

Fig. 5 is an explanatory diagram depicting the results of determining the execution histories depicted in Fig. 2 using the criteria depicted in Fig. 4. For example, the user identified by the user ID "A" executed the scheme identified by the scheme ID "001" continuously for 10 days. The duration threshold for determining the execution of the scheme identified by the scheme ID "001" as successful is 25 days. Accordingly, the execution of the scheme identified by the scheme ID "001" by the user is determined as failed.

The duration criterion may be set by the supervisor or the like beforehand according to past experience and the like, or calculated by the scheme result determination unit 11. For example, the scheme result determination unit 11 may calculate, using the execution histories depicted in Fig. 2, the median value of the execution durations of each scheme, and set the median value as the duration threshold. Alternatively, the scheme result determination unit 11 may calculate the mean value of the execution durations of each scheme, and set the mean value as the duration threshold. In this case, however, the determination is more likely to be affected by lopsided data. The use of the median value as the duration threshold is therefore more preferable.

The combination generation unit 12 generates, for each user, each combination including a scheme determined as failed and a scheme determined as successful, based on the results of the determination by the scheme result determination unit 11. Hereafter, a scheme determined as failed is also referred to as "failed scheme", and a scheme determined as successful as "successful scheme". The combination may include not only information identifying each of the successful scheme and the failed scheme, but also accompanying information of the successful scheme and accompanying information of the failed scheme.

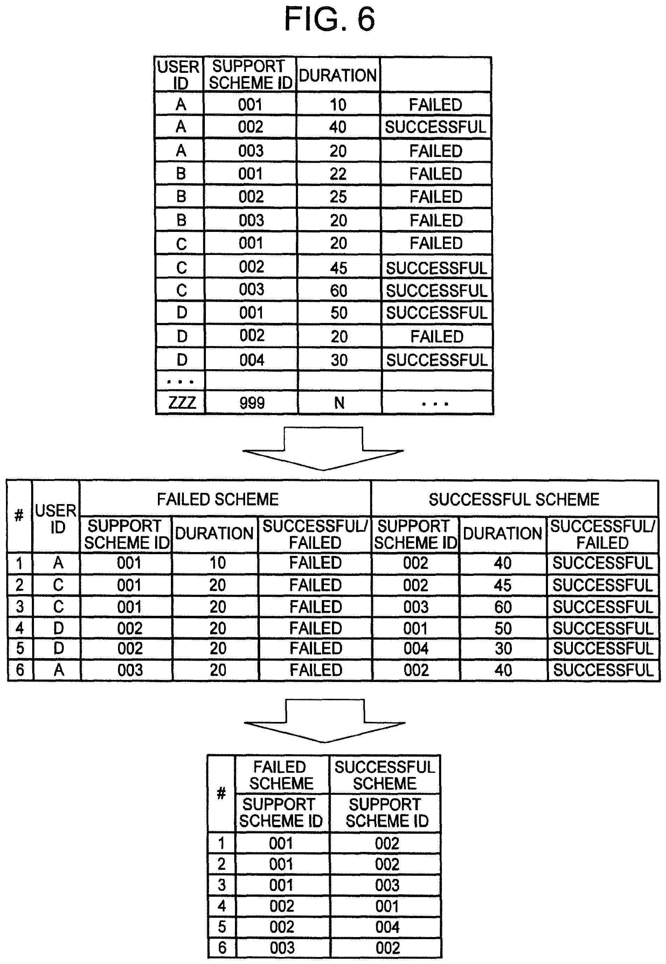

Fig. 6 is an explanatory diagram depicting an example of the process of generating combinations of failed schemes and successful schemes. In the example depicted in Fig. 6, the combination generation unit 12 generates the combinations based on the determination results depicted in Fig. 5. For example, regarding the user identified by the user ID "A", the schemes identified by the support scheme IDs "001" and "003" are failed schemes, and the scheme identified by the support scheme ID "002" is a successful scheme.

Hence, the combination generation unit 12 generates, for the user identified by the user ID "A", the combination of the scheme identified by the support scheme ID "001" and the scheme identified by the support scheme ID "002" and the combination of the scheme identified by the support scheme ID "003" and the scheme identified by the support scheme ID "002". The same applies to the other users.

The combination generation unit 12 may generate overlapping combinations of a failed scheme and a successful scheme. In the example depicted in Fig. 6, the same combinations (the first and second rows) are generated from the execution history of the user identified by the user ID "A" and the execution history of the user identified by the user ID "C". In the case where such overlapping combinations of a failed scheme and a successful scheme are generated, the combination generation unit 12 may keep one combination and delete the other overlapping combination(s).

The scheme candidate storage unit 16 stores the combinations of failed schemes and successful schemes generated by the combination generation unit 12. The scheme candidate storage unit 16 is realized by a magnetic disk as an example.

The scheme input unit 13 receives input of a scheme recognized as failed by the supervisor. The scheme input unit 13 is realized by an input device such as a keyboard or a touch panel for receiving input from the user or the supervisor. In the case of receiving input from the user or the supervisor via a network, the scheme input unit 13 may be realized by a network interface for receiving the input.

The scheme recommendation unit 14 extracts, when the scheme recognized as failed is input, each combination including the failed scheme that matches the input scheme, from the combinations generated by the combination generation unit 12. The scheme recommendation unit 14 extracts the successful scheme included in the extracted combination, and outputs the successful scheme as the recommendation result.

In the case where only one successful scheme corresponds to the failed scheme, the scheme recommendation unit 14 may output the successful scheme as the recommendation result. In the case where a plurality of successful schemes correspond to the failed scheme, the scheme recommendation unit 14 may output all of the successful schemes as the recommendation result, or output one of the successful schemes as the recommendation result based on a predetermined rule.

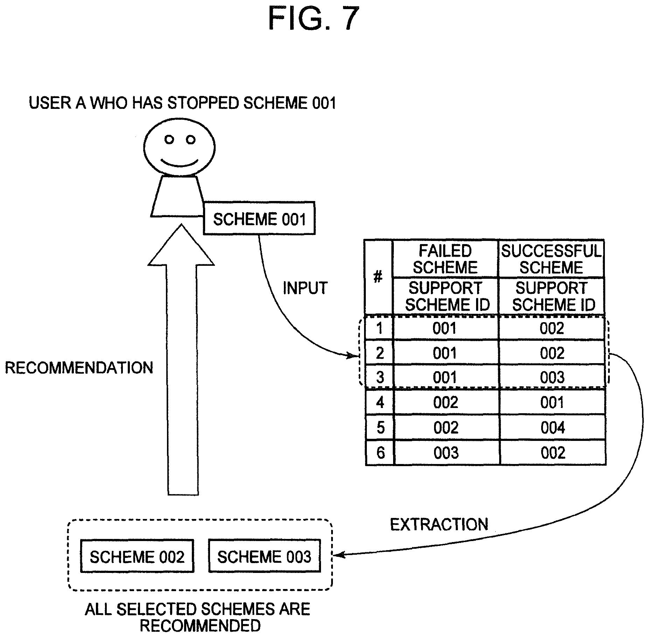

Fig. 7 is an explanatory diagram depicting an example of the process of recommending one or more schemes. For example, when the user A who has failed to execute the scheme identified by the support scheme ID "001" inputs the support scheme ID "001" identifying the failed scheme via the scheme input unit 13, the scheme recommendation unit 14 extracts each combination including the failed scheme identified by the support scheme ID "001", and extracts the support scheme IDs "002" and "003" of the successful schemes included in the extracted combinations. The scheme recommendation unit 14 recommends all of the extracted schemes to the user A. In the example depicted in Fig. 7, the plurality of schemes are retrieved, which may be recommended in any order.

The scheme result determination unit 11, the combination generation unit 12, and the scheme recommendation unit 14 are realized by a CPU of a computer operating according to a program (scheme recommendation program). For example, the program may be stored in a storage unit (not depicted) in the scheme recommendation device 10, with the CPU reading the program and, according to the program, operating as the scheme result determination unit 11, the combination generation unit 12, and the scheme recommendation unit 14. Alternatively, the scheme result determination unit 11, the combination generation unit 12, and the scheme recommendation unit 14 may be each realized by dedicated hardware.

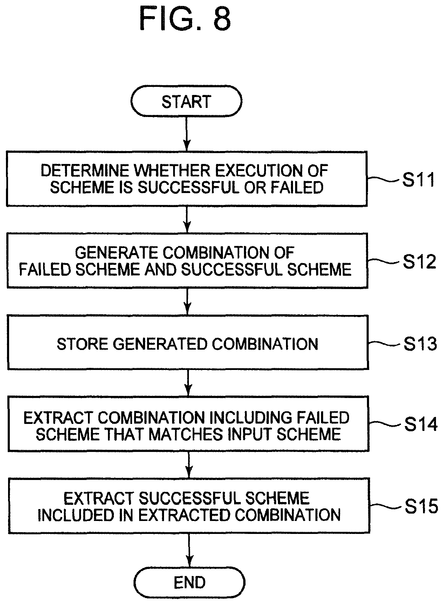

The following describes an example of the operation of the scheme recommendation device 10 in this exemplary embodiment. Fig. 8 is a flowchart depicting an example of the operation of the scheme recommendation device 10 in this exemplary embodiment.

First, the scheme result determination unit 11 determines, for each scheme executed by each user, whether the execution of the scheme by the user is successful or failed, with reference to the scheme histories stored in the scheme history storage unit 20 (step S11). The combination generation unit 12 generates, for each user, each combination including a scheme determined as failed and a scheme determined as successful, based on the results of the determination by the scheme result determination unit 11 (step S12). The combination generation unit 12 stores the generated combinations in the scheme candidate storage unit 16 (step S 13).

Subsequently, when the scheme input unit 13 receives input of a scheme recognized as failed by the supervisor, the scheme recommendation unit 14 extracts each combination including the failed scheme that matches the input scheme, from the combinations generated by the combination generation unit 12 (step S 14). The scheme recommendation unit 14 extracts the successful scheme included in the extracted combination, and outputs the extracted successful scheme as the recommendation result (step S15).

As described above, in this exemplary embodiment, the scheme result determination unit 11 determines, for each scheme executed by each user, whether the execution of the scheme by the user is successful or failed, based on the duration of the execution of the scheme by the user and the duration threshold or range used to determine whether or not the scheme is successful. The combination generation unit 12 generates, for each user, each combination of a failed scheme and a successful scheme. When a scheme recognized as failed is input via the scheme input unit 13, the scheme recommendation unit 14 extracts each combination including the failed scheme that matches the input scheme from the generated combinations, and recommends the successful scheme included in the extracted combination.

It is thus possible to appropriately recommend, to a person who has failed to successfully execute a scheme (i.e. a person who has input a scheme recognized as failed), the next best scheme.

### Exemplary Embodiment 2

The following describes Exemplary Embodiment 2 of a scheme recommendation device according to the present invention. In Exemplary Embodiment 1, the scheme recommendation unit 14 recommends all successful schemes corresponding to the failed scheme, from among the combinations generated by the combination generation unit 12. This exemplary embodiment, on the other hand, describes a method of recommending a successful scheme based on summarized information obtained by summarizing the combinations generated by the combination generation unit 12 so that a more suitable successful scheme can be recommended.

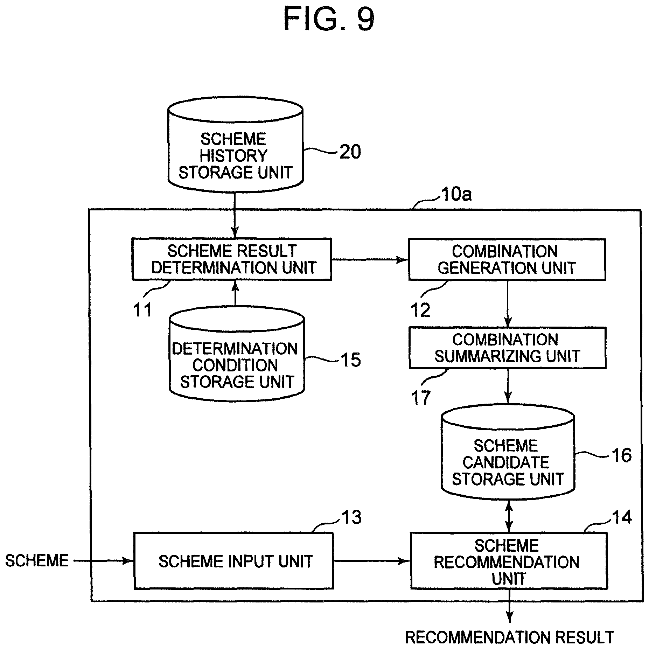

Fig. 9 is a block diagram depicting an example of the structure of Exemplary Embodiment 2 of a scheme recommendation device according to the present invention. The same components as those in Exemplary Embodiment 1 are given the same reference signs as in Fig. 1, and their description is omitted. A scheme recommendation device 10a in this exemplary embodiment includes the scheme result determination unit 11, the combination generation unit 12, the scheme input unit 13, the scheme recommendation unit 14, the determination condition storage unit 15, the scheme candidate storage unit 16, and a combination summarizing unit 17.

The scheme recommendation device in this exemplary embodiment differs from that in Exemplary Embodiment 1 in that the combination summarizing unit 17 is further included. Note that the combination generation unit 12 may perform the process of the combination summarizing unit 17.

The combination summarizing unit 17 generates summarized information by summarizing the combinations generated by the combination generation unit 12. The combination summarizing unit 17 is realized by a CPU of a computer operating according to a program (scheme recommendation program). In this case, the scheme recommendation unit 14 extracts summarized information including the failed scheme that matches the input scheme, and recommends the successful scheme using the extracted summarized information. In detail, the scheme recommendation unit 14 may output at least one piece of summarized information together with the recommended successful scheme.

The scheme recommendation unit 14 may specify summarized information determined to be recommended with higher priority, and select and recommend the successful scheme corresponding to the specified summarized information. Here, the scheme recommendation unit 14 may use only one piece of summarized information, or use a plurality of pieces of summarized information. The scheme recommendation unit 14 may output the summarized information together with the recommended successful scheme.

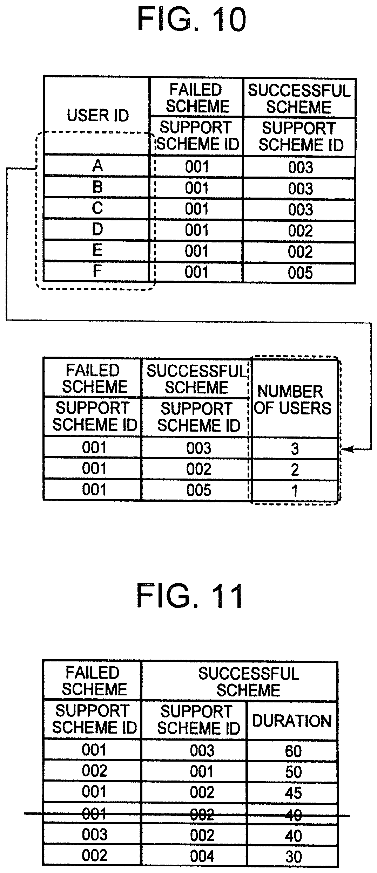

The following describes specific examples of the summarized information generated by the combination summarizing unit 17. Fig. 10 is an explanatory diagram depicting a first example of the process of summarizing combinations. In the example depicted in Fig. 10, the combination summarizing unit 17 counts the number of users each corresponding to the scheme history based on which a combination is generated, to generate summarized information. Here, the scheme recommendation unit 14 may output the number of users as the summarized information, together with the recommended successful scheme.

In this case, it can be considered that a successful scheme corresponding to summarized information with a larger number of users is to be recommended with higher priority. Hence, the scheme recommendation unit 14 may recommend the successful schemes in descending order of the number of users or recommend the successful scheme with the largest number of users, from among the extracted summarized information.

In the example depicted in Fig. 10, the scheme recommendation unit 14 may recommend the successful schemes in descending order of the number of users, i.e. in the order of the scheme identified by the support scheme ID "003", the scheme identified by the support scheme ID "002", and the scheme identified by the support scheme ID "005". Such a process enables the user or the supervisor to select a scheme successfully executed by more users in the past.

Fig. 11 is an explanatory diagram depicting a second example of the process of summarizing combinations. In the example depicted in Fig. 11, the combination summarizing unit 17 assigns priorities to (i.e. sorts) the combinations in descending order of the duration of the included successful scheme. In the case where there are overlapping combinations of a failed scheme and a successful scheme, the combination summarizing unit 17 may keep only a combination with a longer duration.

Here, the scheme recommendation unit 14 may output the duration as the summarized information, together with the recommended successful scheme. In this case, it can be considered that a successful scheme corresponding to summarized information with a longer duration is to be recommended with higher priority. Hence, the scheme recommendation unit 14 may recommend the successful schemes in descending order of the duration or recommend the successful scheme with the longest duration, from among the extracted summarized information. Such a process enables the user or the supervisor to select a scheme executed for a longer duration from past successful examples.

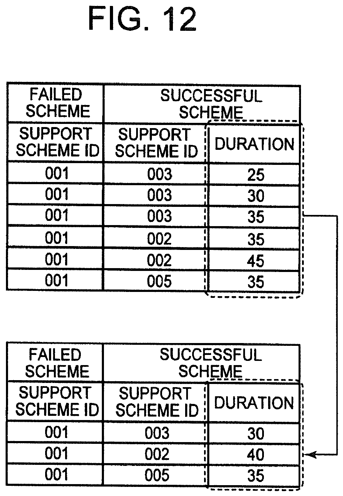

Fig. 12 is an explanatory diagram depicting a third example of the process of summarizing combinations. In the example depicted in Fig. 12, the combination summarizing unit 17 determines, for the same combination of a failed scheme and a successful scheme, a period applied as the duration of the successful scheme included in the combination. The combination summarizing unit 17 may use the mean value, median value, sum, or the like of the durations as the applied period. In the example depicted in Fig. 12, the mean value of the durations is calculated as the period applied as the duration.

Here, the scheme recommendation unit 14 may output the applied period as the summarized information, together with the recommended successful scheme. In this case, it can be considered that a successful scheme corresponding to summarized information with a longer applied period is to be recommended with higher priority. Hence, the scheme recommendation unit 14 may recommend the successful schemes in descending order of the applied period or recommend the successful scheme with the longest applied period, from among the extracted summarized information. Such a process enables the user or the supervisor to select a scheme having statistically higher continuity.

The summarized information generated by the combination summarizing unit 17 is not limited to the aforementioned summarized information. Moreover, the combination summarizing unit 17 may generate a plurality of types of summarized information. Here, the scheme recommendation unit 14 may recommend a successful scheme by combining the plurality of types of summarized information. An example of the method of combining a plurality of types of summarized information is the following: The priority of applying summarized information is set beforehand, and the scheme recommendation unit 14 recommends a successful scheme according to the priority. With the use of a plurality of types of summarized information, a more suitable scheme can be recommended depending on the situation.

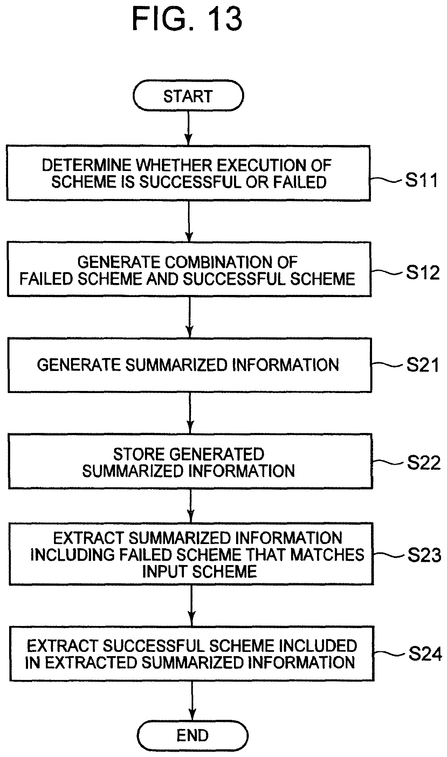

The following describes an example of the operation of the scheme recommendation device 10a in this exemplary embodiment. Fig. 13 is a flowchart depicting an example of the operation of the scheme recommendation device 10a in this exemplary embodiment. The process in which the scheme result determination unit 11 determines, for each scheme executed by each user, whether the execution of the scheme by the user is successful or failed and the combination generation unit 12 generates, for each user, each combination of a failed scheme and a successful scheme is the same as the process of steps S11 and S 12 in Fig. 8.

The combination summarizing unit 17 generates summarized information by summarizing the combinations generated by the combination generation unit 12 (step S21). The combination summarizing unit 17 stores the generated summarized information in the scheme candidate storage unit 16 (step S22).

Subsequently, when the scheme input unit 13 receives input of a scheme recognized as failed by the supervisor, the scheme recommendation unit 14 extracts summarized information including the failed scheme that matches the input scheme, from the summarized information generated by the combination summarizing unit 17 (step S23). The scheme recommendation unit 14 extracts the successful scheme included in the extracted summarized information, and recommends the successful scheme using the extracted summarized information (step S24).

As described above, in this exemplary embodiment, the combination summarizing unit 17 generates summarized information by summarizing the combinations generated by the combination generation unit 12. The scheme recommendation unit 14 extracts summarized information including the failed scheme that matches the input scheme, and recommends the successful scheme using the extracted summarized information. Here, the scheme recommendation unit 14 may output the summarized information, or recommend the successful scheme according to priority.

In detail, the combination summarizing unit 17 may count the number of users each corresponding to the scheme history based on which the combination is generated to generate summarized information, so that the scheme recommendation unit 14 recommends a successful scheme with a larger number of users from among the summarized information including the failed scheme that matches the input scheme.

Alternatively, the combination summarizing unit 17 may summarize the combinations in descending order of the duration of the included successful scheme, so that the scheme recommendation unit 14 recommends a successful scheme with a longer duration from among the summarized information including the failed scheme that matches the input scheme.

Alternatively, the combination summarizing unit 17 may determine, for the same combination of a failed scheme and a successful scheme, a period applied as the duration of the successful scheme included in the combination, so that the scheme recommendation unit 14 recommends a successful scheme with a longer applied period from among the summarized information including the failed scheme that matches the input scheme.

With such structures, it is possible to recommend a more appropriate successful scheme to the user or the supervisor, in addition to the advantageous effects of Exemplary Embodiment 1.

### Exemplary Embodiment 3

The following describes Exemplary Embodiment 3 of a scheme recommendation device according to the present invention. In Exemplary Embodiment 1, the scheme recommendation unit 14 recommends all successful schemes corresponding to the failed scheme, from among the combinations generated by the combination generation unit 12. In the exemplary embodiments described below (Exemplary Embodiments 3 and 4), the scheme recommendation unit 14 performs a process of, for each extracted combination, specifying information (hereafter referred to as "priority information") used to determine a successful scheme to be recommended with priority. The scheme recommendation unit 14 recommends a successful scheme using the specified priority information.

In Exemplary Embodiment 3, the scheme recommendation unit 14 recommends a successful scheme based on the category in which the scheme is classified. The following describes a method of recommending a successful scheme based on the category in which the scheme is classified so that a more suitable successful scheme can be recommended.

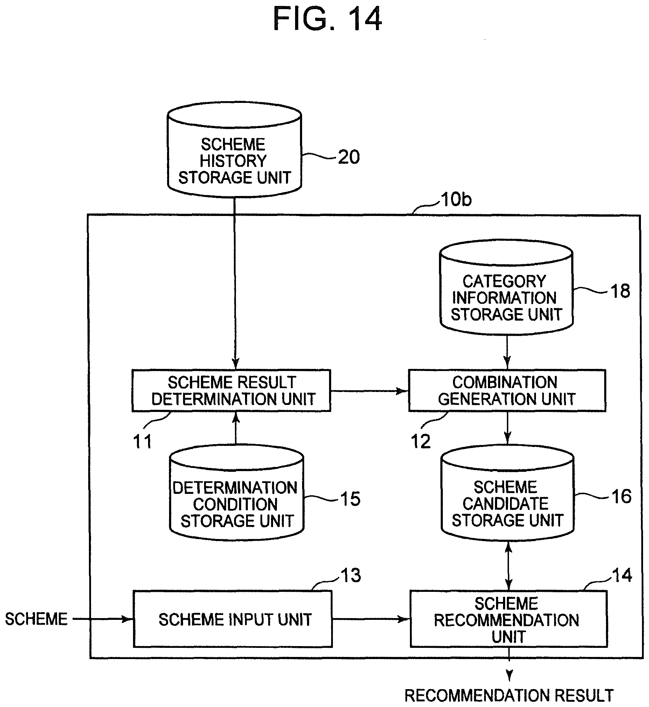

Fig. 14 is a block diagram depicting an example of the structure of Exemplary Embodiment 3 of a scheme recommendation device according to the present invention. The same components as those in Exemplary Embodiment 1 are given the same reference signs as in Fig. 1, and their description is omitted. A scheme recommendation device 10b in this exemplary embodiment includes the scheme result determination unit 11, the combination generation unit 12, the scheme input unit 13, the scheme recommendation unit 14, the determination condition storage unit 15, the scheme candidate storage unit 16, and a category information storage unit 18.

The scheme recommendation device in this exemplary embodiment differs from that in Exemplary Embodiment 1 in that the category information storage unit 18 is further included. The scheme recommendation device 10b in this exemplary embodiment may include the combination summarizing unit 17 in Exemplary Embodiment 2.

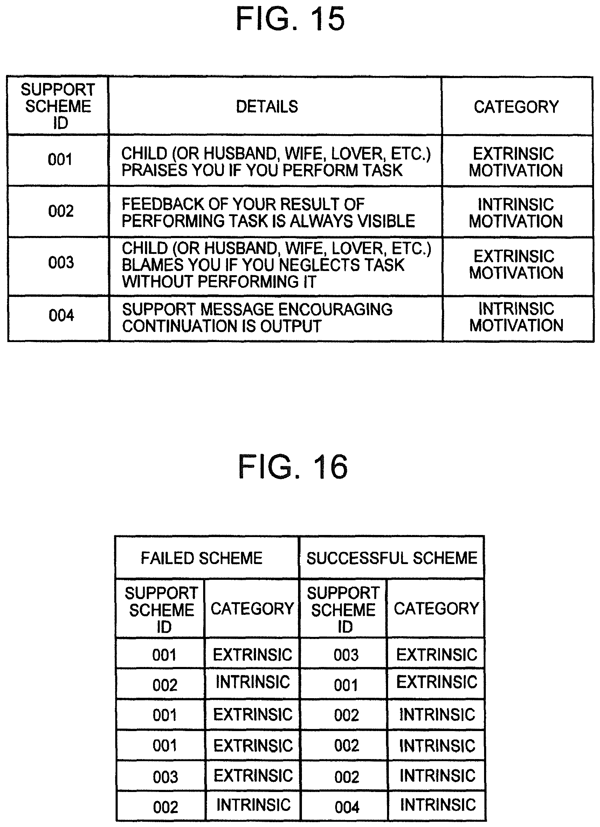

The category information storage unit 18 stores the categories in which the schemes are classified. Fig. 15 is an explanatory diagram depicting an example of the categories stored in the category information storage unit 18. In the example depicted in Fig. 15, the schemes identified by the support scheme IDs "001" and "003" are based on extrinsic motivation, whereas the schemes identified by the support scheme IDs "002" and "004" are based on intrinsic motivation.

The categories used for scheme classification are not limited to the aforementioned categories. Other examples of the categories include: schemes intended for specified users and schemes intended for many and unspecified users; and schemes subjected to qualitative evaluation and schemes subjected to quantitative evaluation.

The category information storage unit 18 also stores the priority of the category of a successful scheme defined for the category of a failed scheme. For example, the intrinsic category may be given higher priority for the extrinsic category. Likewise, the extrinsic category may be given higher priority for the intrinsic category. Such priority is, however, merely an example. For instance, the relationship between schemes intended for specified users and schemes intended for many and unspecified users and/or the relationship between schemes subjected to qualitative evaluation and schemes subjected to quantitative evaluation may be defined as with the aforementioned priority.

The scheme recommendation unit 14 recommends a successful scheme from among the successful schemes included in the extracted combinations, according to the category priority stored in the category information storage unit 18. In detail, the scheme recommendation unit 14 specifies, for each extracted combination, the priority of the category of the successful scheme defined for the category of the failed scheme as priority information, and recommends the successful scheme using the specified priority information.

Here, the scheme recommendation unit 14 may output the priority information together with the recommended successful scheme. Any number of pieces of information or any type of information may be output. The scheme recommendation unit 14 may select and recommend a successful scheme to be recommended with higher priority. The scheme recommendation unit 14 may recommend a successful scheme using only one piece of priority information, or recommend a successful scheme using a plurality of pieces of priority information.

Fig. 16 is an explanatory diagram depicting an example of adding the categories to the combinations of successful schemes and failed schemes. The scheme recommendation unit 14 may specify the combinations depicted in Fig. 16, using the combinations generated by the combination generation unit 12 and the category information stored in the category information storage unit 18. Alternatively, the combination generation unit 12 may generate the combinations depicted in Fig. 16, with reference to the category information storage unit 18.

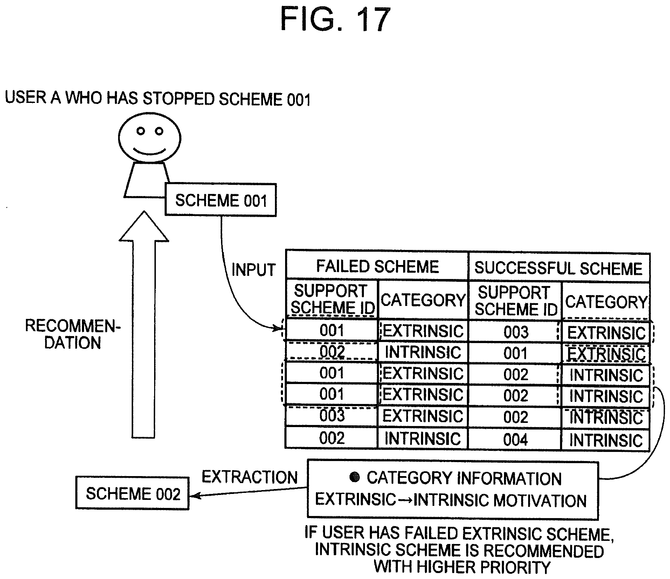

Fig. 17 is an explanatory diagram depicting another example of recommending one or more schemes. For example, when the user A who has failed to execute the scheme identified by the support scheme ID "001" inputs the support scheme ID "001" identifying the failed scheme via the scheme input unit 13, the scheme recommendation unit 14 extracts each combination including the failed scheme identified by the support scheme ID "001 ", and extracts the support scheme IDs "002" and "003" of the successful schemes included in the extracted combinations.

Here, the scheme identified by the support scheme ID "001" is classified in the extrinsic category, and the category priority indicating that the intrinsic category is recommended with priority in the case where the failed scheme is classified in the extrinsic category is defined. Accordingly, the scheme recommendation unit 14 may recommend the scheme identified by the support scheme ID "002", which is classified in the intrinsic category, to the user A.

As described above, in this exemplary embodiment, the scheme recommendation unit 14 specifies the priority information of each extracted combination, based on the priority of the category of a successful scheme defined for the category of a failed scheme. In detail, the scheme recommendation unit 14 recommends, from among the successful schemes included in the extracted combinations, a successful scheme in the category with higher priority defined for the category of the failed scheme. This is because a scheme classified in the same category as the failed scheme is more likely to be failed even if recommended as the next scheme. According to this exemplary embodiment, the category priority defined based on the causal relationship between schemes, etc. is used for recommendation. It is thus possible to recommend a more appropriate successful scheme to the user or the supervisor, in addition to the advantageous effects of Exemplary Embodiment 1.

### Exemplary Embodiment 4

The following describes Exemplary Embodiment 4 of a scheme recommendation device according to the present invention. In Exemplary Embodiment 1, the scheme recommendation unit 14 recommends all successful schemes corresponding to the failed scheme, from among the combinations generated by the combination generation unit 12. This exemplary embodiment, on the other hand, describes a method of recommending a successful scheme in consideration of the attribute of the user corresponding to the scheme history based on which the combination is generated so that a more suitable successful scheme can be recommended. In this exemplary embodiment, too, the scheme recommendation unit 14 performs the process of specifying priority information for each extracted combination, and recommends a successful scheme using the specified priority information.

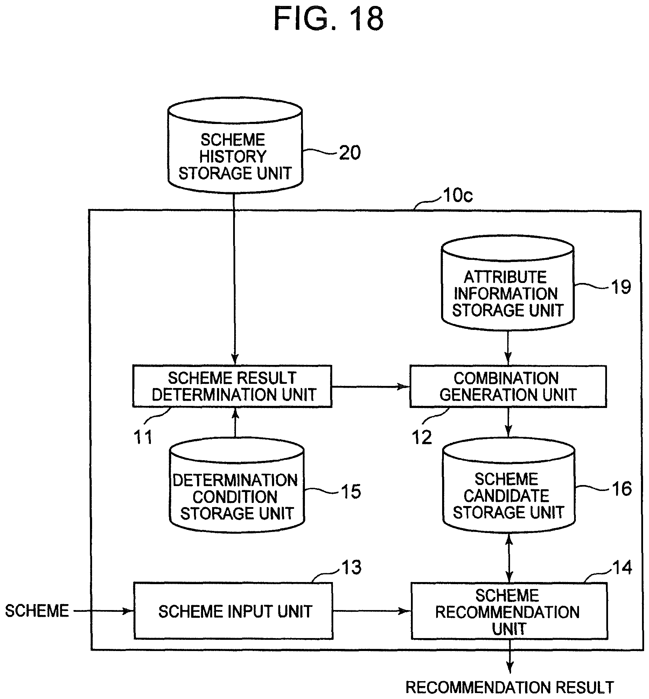

Fig. 18 is a block diagram depicting an example of the structure of Exemplary Embodiment 4 of a scheme recommendation device according to the present invention. The same components as those in Exemplary Embodiment 1 are given the same reference signs as in Fig. 1, and their description is omitted. A scheme recommendation device 10c in this exemplary embodiment includes the scheme result determination unit 11, the combination generation unit 12, the scheme input unit 13, the scheme recommendation unit 14, the determination condition storage unit 15, the scheme candidate storage unit 16, and an attribute information storage unit 19.

The scheme recommendation device in this exemplary embodiment differs from that in Exemplary Embodiment 1 in that the attribute information storage unit 19 is further included. The scheme recommendation device 10c in this exemplary embodiment may include the combination summarizing unit 17 in Exemplary Embodiment 2, and may include the category information storage unit 18 in Exemplary Embodiment 3.

The attribute information storage unit 19 stores the attribute information of each user. The attribute information in this exemplary embodiment includes not only the user's nature or features but also the environment surrounding the user, etc. Examples of the attribute information include sex, age, household composition, smoking/non-smoking, hospital visit history, exercise history, eating behavior, dietary preference, past failed schemes and their durations, and past successful schemes and their durations.

The combination generation unit 12 adds, to each combination of a failed scheme and a successful scheme, the attribute information of the user corresponding to the scheme history based on which the combination is generated. In detail, when generating a combination of a failed scheme and a successful scheme for each user, the combination generation unit 12 acquires the attribute information of the user with reference to the attribute information storage unit 19, and adds the attribute information to the combination. The combination generation unit 12 stores the generated combinations in the scheme candidate storage unit 16.

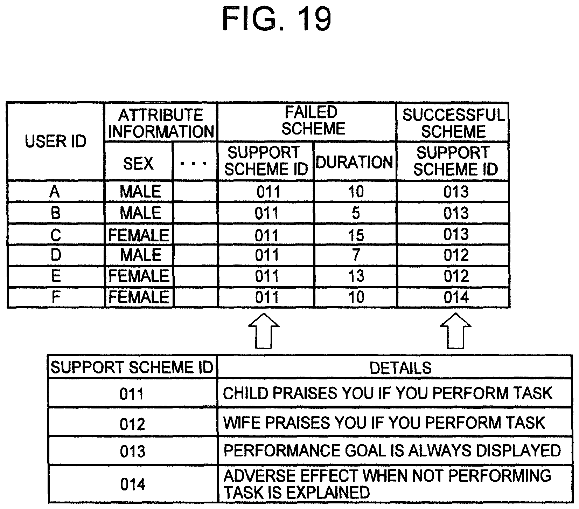

Fig. 19 is an explanatory diagram depicting an example of the combinations to which the attribute information has been added. In the example depicted in Fig. 19, the attribute information including the user's sex is added to each combination.

The scheme recommendation unit 14, when a scheme recognized as failed and the identification information of the user to whom the recommendation is directed are input, specifies the degree of similarity to the attribute information of the user identified by the input identification information as priority information, for each extracted combination including the failed scheme that matches the input scheme. The scheme recommendation unit 14 recommends a successful scheme using the specified priority information. For example, the scheme recommendation unit 14 recommends a successful scheme included in a combination with a higher degree of similarity to the attribute information of the user identified by the input identification information.

In detail, when the scheme recognized as failed and the identification information of the user to whom the recommendation is directed are input via the scheme input unit 13, the scheme recommendation unit 14 acquires the attribute information of the user with reference to the attribute information storage unit 19. The attribute information may be input together with the identification information of the user via the scheme input unit 13. In this case, the scheme recommendation unit 14 may use the input attribute information.

The scheme recommendation unit 14 in this exemplary embodiment may also output the priority information together with the recommended successful scheme. Any number of pieces of information or any type of information may be output. The scheme recommendation unit 14 may select and recommend a successful scheme to be recommended with higher priority. The scheme recommendation unit 14 may recommend a successful scheme using only one piece of priority information, or recommend a successful scheme using a plurality of pieces of priority information.

The scheme recommendation unit 14 may determine that the degree of similarity is higher when a larger number of attributes match between the attribute information of the user and the attribute information added to the generated combination. The scheme recommendation unit 14 may weight each attribute, and determine that the degree of similarity is higher when the total weight of matching attributes is larger. The scheme recommendation unit 14 may determine that the degree of similarity is higher when the duration of the failed scheme and the duration of the failed scheme added to the generated combination are closer to each other.

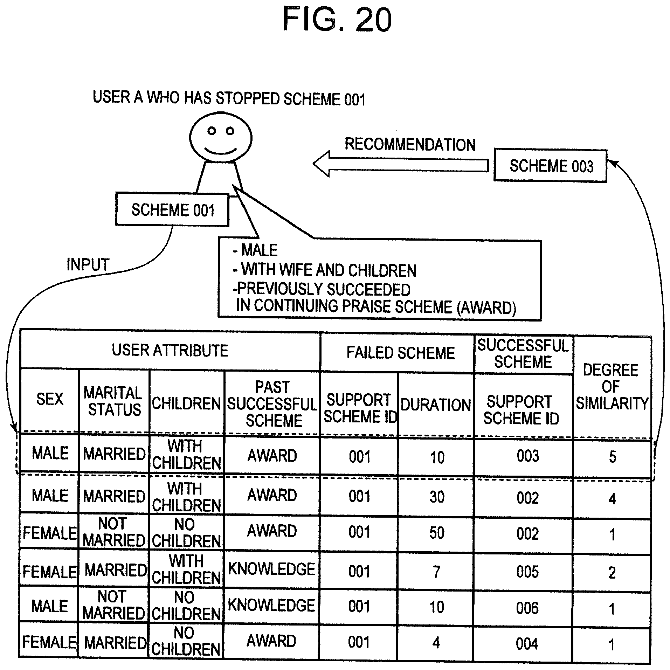

Fig. 20 is an explanatory diagram depicting another example of recommending one or more schemes. For example, when the user A who has failed to execute the scheme identified by the support scheme ID "001" inputs the support scheme ID "001" identifying the failed scheme via the scheme input unit 13, the scheme recommendation unit 14 acquires the attribute information of the user A from the attribute information storage unit 19.

In the example depicted in Fig. 20, the information such as "male", "with wife and children (married with children)", and "previously succeeded in continuing praise scheme (award)" are acquired as the attributes of the user A.

The scheme recommendation unit 14 recommends, from among each combination including the failed scheme that matches the input support scheme ID "001", a successful scheme included in a combination with the highest degree of similarity to the attribute information of the user. In the example depicted in Fig. 20, the degree of similarity of the combination in the first row is 5 which is higher than the degrees of similarity of the other combinations, as a result of similarity determination. Hence, the scheme recommendation unit 14 may recommend the scheme identified by the support scheme ID "003" to the user A, as the successful scheme included in the combination determined to have the highest degree of similarity.

As described above, in this exemplary embodiment, the combination generation unit 12 adds, to each combination of a failed scheme and a successful scheme, the attribute information of the user corresponding to the scheme history based on which the combination is generated. The scheme recommendation unit 14, when a scheme recognized as failed and the identification information of the user to whom the recommendation is directed are input, specifies the priority information of each combination including the failed scheme that matches the input scheme, based on the degree of similarity to the attribute information of the user identified by the input identification information. In detail, the scheme recommendation unit 14 recommends a successful scheme included in a combination with a higher degree of similarity to the attribute information of the user identified by the input identification information.

Of successful schemes executed by users in the past, a successful scheme executed by a user is more likely to be successfully executed by another user having similar attributes. In this exemplary embodiment, a successful scheme executed by a user having similar attributes in the past is recommended with priority. It is thus possible to recommend a more appropriate successful scheme, in addition to the advantageous effects of Exemplary Embodiment 1. This enables the user to select a past successful scheme of another similar user.

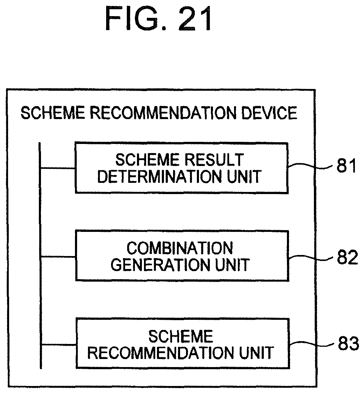

An overview of the present invention is described below. Fig. 21 is a block diagram schematically depicting a scheme recommendation device according to the present invention. The scheme recommendation device according to the present invention includes: a scheme result determination unit 81 (e.g. the scheme result determination unit 11) which determines, for each scheme executed by each user, whether the execution of the scheme by the user is successful or failed, based on a duration of the execution of the scheme by the user and a duration threshold or range used to determine whether or not the scheme is successful (e.g. based on an execution history stored in the scheme history storage unit 20); a combination generation unit 82 (e.g. the combination generation unit 12) which generates, for each user, a combination including a failed scheme and a successful scheme based on a result of the determination, the failed scheme being a scheme determined as failed, and the successful scheme being a scheme determined as successful; and a scheme recommendation unit 83 (e.g. the scheme recommendation unit 14), when a scheme recognized as failed is input, which extracts a combination including the failed scheme that matches the input scheme from generated combinations, and recommends the successful scheme included in the extracted combination.

With such a structure, it is possible to appropriately recommend, to a person who has failed to successfully execute a scheme, the next best scheme.

The scheme recommendation unit 83 may specify, for each extracted combination, priority information (e.g. category priority, attribute information similarity) used to determine the successful scheme to be recommended with priority, and recommend the successful scheme using the specified priority information.

In detail, the scheme recommendation unit 83 may specify, for each extracted combination, the priority information based on a priority of a category of a successful scheme defined for a category of the failed scheme. With such a structure, the category priority defined based on the causal relationship between schemes, etc. is used for recommendation, so that a more appropriate successful scheme can be recommended to the user or the supervisor.

The combination generation unit 82 may add, to each combination of the failed scheme and the successful scheme, attribute information of a user corresponding to a scheme history based on which the combination is generated, and when the scheme recognized as failed and identification information of a user to whom the recommendation is directed are input, the scheme recommendation unit 83 may specify the priority information of the combination including the failed scheme that matches the input scheme, based on a degree of similarity to attribute information of the user identified by the input identification information. With such a structure, a successful scheme executed by a user having similar attributes in the past is recommended with priority, so that a more appropriate successful scheme can be recommended.

When recommending the successful scheme, the scheme recommendation unit 83 may output at least one piece of priority information together with the successful scheme. The scheme recommendation unit 83 may select and recommend the successful scheme to be recommended with higher priority, based on at least one piece of priority information.

The scheme recommendation device may include a combination summarizing unit (e.g. the combination summarizing unit 17) which generates summarized information (e.g. information summarized based on the number of users, the duration, etc.) by summarizing the combinations generated by the combination generation unit 82, and the scheme recommendation unit 83 may extract summarized information including the failed scheme that matches the input scheme, and recommend the successful scheme using the extracted summarized information.

When recommending the successful scheme, the scheme recommendation unit 83 may output at least one piece of summarized information together with the successful scheme. The scheme recommendation unit 83 may select and recommend the successful scheme corresponding to summarized information (e.g. with a larger number of users or a longer duration) determined to be recommended with higher priority, based on at least one piece of summarized information.

In detail, the combination summarizing unit may generate the summarized information by counting the number of users each corresponding to a scheme history based on which a combination is generated. The combination summarizing unit may summarize the combinations in descending order of a duration of a successful scheme included in a combination. The combination summarizing unit may determine, for the same combination of a failed scheme and a successful scheme, a period applied as a duration of the successful scheme included in the combination.

While the present invention has been described with reference to the above exemplary embodiments and examples, the present invention is not limited to the above exemplary embodiments and examples. Various changes understandable by those skilled in the art can be made to the structures and details of the present invention within the scope of the present invention.

This application claims priority based on Japanese Patent Application No. 2014-055840 filed on March 19, 2014, the disclosure of which is incorporated herein in its entirety.

### Reference Signs List

- 10, 10a, 10b, 10c: scheme recommendation device
- 11: scheme result determination unit
- 12: combination generation unit
- 13: scheme input unit
- 14: scheme recommendation unit
- 15: determination condition storage unit
- 16: scheme candidate storage unit
- 17: combination summarizing unit
- 18: category information storage unit
- 19: attribute information storage unit
- 20: scheme history storage unit

## Claims

1. A scheme recommendation device comprising:
a scheme result determination unit which determines, for each scheme executed by each user, whether the execution of the scheme by the user is successful or failed, based on a duration of the execution of the scheme by the user and a duration threshold or range used to determine whether or not the scheme is successful;
a combination generation unit which generates, for each user, a combination including a failed scheme and a successful scheme based on a result of the determination, the failed scheme being a scheme determined as failed, and the successful scheme being a scheme determined as successful; and
a scheme recommendation unit, when a scheme recognized as failed is input, which extracts a combination including the failed scheme that matches the input scheme from generated combinations, and recommends the successful scheme included in the extracted combination.

2. The scheme recommendation device according to claim 1, wherein the scheme recommendation unit specifies, for each extracted combination, priority information used to determine the successful scheme to be recommended with priority, and recommends the successful scheme using the specified priority information.

3. The scheme recommendation device according to claim 2, wherein the scheme recommendation unit specifies, for each extracted combination, the priority information based on a priority of a category of a successful scheme defined for a category of the failed scheme.

4. The scheme recommendation device according to claim 2 or 3, wherein the combination generation unit adds, to each combination of the failed scheme and the successful scheme, attribute information of a user corresponding to a scheme history based on which the combination is generated, and
wherein when the scheme recognized as failed and identification information of a user to whom the recommendation is directed are input, the scheme recommendation unit specifies the priority information of the combination including the failed scheme that matches the input scheme, based on a degree of similarity to attribute information of the user identified by the input identification information.

5. The scheme recommendation device according to any one of claims 2 to 4, wherein when recommending the successful scheme, the scheme recommendation unit outputs at least one piece of priority information together with the successful scheme.

6. The scheme recommendation device according to any one of claims 2 to 5, wherein the scheme recommendation unit selects and recommends the successful scheme to be recommended with higher priority, based on at least one piece of priority information.

7. The scheme recommendation device according to any one of claims 1 to 6, comprising
a combination summarizing unit which generates summarized information by summarizing the combinations generated by the combination generation unit,
wherein the scheme recommendation unit extracts summarized information including the failed scheme that matches the input scheme, and recommends the successful scheme using the extracted summarized information.

8. The scheme recommendation device according to claim 7, wherein when recommending the successful scheme, the scheme recommendation unit outputs at least one piece of summarized information together with the successful scheme.

9. The scheme recommendation device according to claim 7 or 8, wherein the scheme recommendation unit selects and recommends the successful scheme corresponding to summarized information determined to be recommended with higher priority, based on at least one piece of summarized information.

10. The scheme recommendation device according to any one of claims 7 to 9, wherein the combination summarizing unit generates the summarized information by counting the number of users each corresponding to a scheme history based on which a combination is generated.

11. The scheme recommendation device according to any one of claims 7 to 10, wherein the combination summarizing unit summarizes the combinations in descending order of a duration of a successful scheme included in a combination.

12. The scheme recommendation device according to any one of claims 7 to 11, wherein the combination summarizing unit determines, for the same combination of a failed scheme and a successful scheme, a period applied as a duration of the successful scheme included in the combination.

13. A scheme recommendation method performed by a scheme recommendation device, the scheme recommendation method comprising:
determining, for each scheme executed by each user, whether the execution of the scheme by the user is successful or failed, based on a duration of the execution of the scheme by the user and a duration threshold or range used to determine whether or not the scheme is successful;
generating, for each user, a combination including a failed scheme and a successful scheme based on a result of the determination, the failed scheme being a scheme determined as failed, and the successful scheme being a scheme determined as successful; and
extracting, when a scheme recognized as failed is input, a combination including the failed scheme that matches the input scheme from generated combinations, and recommending the successful scheme included in the extracted combination.

14. The scheme recommendation method according to claim 13, comprising
specifying, for each extracted combination, priority information used to determine the successful scheme to be recommended with priority, and recommending the successful scheme using the specified priority information.

15. A scheme recommendation program for causing a computer to execute:
a scheme result determination process of determining, for each scheme executed by each user, whether the execution of the scheme by the user is successful or failed, based on a duration of the execution of the scheme by the user and a duration threshold or range used to determine whether or not the scheme is successful;
a combination generation process of generating, for each user, a combination including a failed scheme and a successful scheme based on a result of the determination, the failed scheme being a scheme determined as failed, and the successful scheme being a scheme determined as successful; and
a scheme recommendation process of, when a scheme recognized as failed is input, extracting a combination including the failed scheme that matches the input scheme from generated combinations, and recommending the successful scheme included in the extracted combination.

16. The scheme recommendation program according to claim 15, causing the computer to, in the scheme recommendation process,
specify, for each extracted combination, priority information used to determine the successful scheme to be recommended with priority, and recommend the successful scheme using the specified priority information.
